# EUROPEAN PATENT APPLICATION

(11) **EP 1 813 312 A2**
(43) Date of publication of application: **01.08.2007**
(21) Application number: 07106162.6
(22) Date of filing: 01.09.2003
(51) Int. Cl.: A61Q 19/02, A61K 8/368, A61K 8/34, A61K 8/63, A61K 8/67

(54) **Depigmenting composition for the skin comprising adapalene and at least one depigmenting agent**

(30) Priority: 05.09.2002 FR 0211022
(62) Divisional of application: 03750634.2
(71) Applicant: GALDERMA RESEARCH & DEVELOPMENT, 06410 Biot (FR)
(72) Inventor: Pelisson, Isabelle, 06220, VALLAURIS (FR); Jomard, André, 06460, SAINT VALLIER DE THIEY (FR)
(74) Representative: Andral, Christophe André Louis

(57) **Abstract**

The invention relates to a depigmenting composition for the skin comprising, in a physiologically acceptable medium, adapalene and at least one depigmenting agent and its pharmaceutical or cosmetic use.

## Description

The invention relates to a depigmenting composition for the skin comprising, in a physiologically acceptable medium, adapalene (6-[3-(1-adamantyl)-4-methoxyphenyl]-2-naphthanoic acid) and at least one depigmenting agent and its pharmaceutical or cosmetic use. The composition according to the invention may also contain a sunscreen.

A. M. Kligman describes, in patent US 3,856,934, a depigmenting composition comprising hydroquinone, retinoic acid and a corticosteroid. This type of combination allows good depigmentation of the skin but causes substantial undesirable effects such as irritation and itching.

Retinoic acid (tretinoin) is known to have on its own a skin depigmenting activity (Guevara I. A. and Pandya A. G. Int. J. Dermatol. 40, 210-215 (2001) unlike adapalene which has no depigmenting activity as is shown in Example 6 below. By virtue of its lack of depigmenting activity in particular, nothing therefore encouraged persons skilled in the art to combine it with a depigmenting agent in a depigmenting composition containing a sunscreen or not.

However, the applicant has discovered, surprisingly, that the combination of adapalene and a depigmenting agent made it possible to obtain a much more rapid depigmenting response than that obtained with the depigmenting agent alone.

The invention therefore relates to a depigmenting composition for the skin comprising, in a physiologically acceptable medium, adapalene and at least one depigmenting agent.

The expression physiologically acceptable medium is understood to mean a medium compatible with the skin, the mucous membranes and/or the superficial body growths.

The expression depigmenting agent is understood to mean any active agent having a skin depigmenting activity. This activity makes it possible to reduce the already existing pigmentation of the skin and also to prevent any additional pigmentation above the natural pigmentation.

There may be mentioned, as depigmenting agent, by way of nonlimiting example, phenolic derivatives, such as hydroquinone, hydroquinone monoethyl ether, hydroquinone monobenzyl ether or 4-hydroxyanisole; kojic acid and its derivatives; azelaic acid and its derivatives; linoleic acid; resorcinol and its derivatives; ellagic acid; hydroxy acids such as glycolic acid; ascorbic acid and its derivatives, in particular ascorbyl glucoside; zinc peroxide; and mercury chloride, arbutin and its derivatives such as those described in applications EP-895 779 and EP-524 109; aminophenol derivatives such as those described in applications WO 99/10318 and WO 99/32077, and in particular N-cholesteryloxy-carbonyl-para-aminophenol and N-ethyloxycarbonyl-para-aminophenol; iminophenol derivatives, in particular those described in application WO 99/22707; L-2-oxothiazolidine-4-carboxylic acid or procysteine, and its salts and esters; and extracts of plants, in particular of liquorice, mulberry and skullcap.

In particular, the depigmenting composition for the skin can comprise, as depigmenting agent, a phenolic derivative, in particular hydroquinone or 4-hydroxyanisole.

The invention also relates to a depigmenting composition for the skin comprising, in a physiologically acceptable medium, adapalene, at least one depigmenting agent and at least one sunscreen.

To give an order of magnitude, the composition according to the invention advantageously comprises between 0.0001 and 20% by weight of adapalene relative to the total weight of the composition and between 0.0001 and 20% by weight of depigmenting agent relative to the total weight of the composition, and preferably, respectively, between 0.001 and 10% by weight of adapalene relative to the total weight of the composition and between 0.025 and 10% by weight of depigmenting agent relative to the total weight of the composition.

The composition may also comprise at least one sunscreen in preferential concentrations ranging from 0.001 to 30.00% by weight relative to the total weight of the composition.

Among the sunscreens, there may be mentioned, by way of nonlimiting example, physical sunscreens such as titanium dioxide, zinc oxide, and chemical sunscreens such as octocrylene, ethylhexyl methoxycinnamate, octyl salicylate, avobenzone, oxybenzone, ecamsule or drometrizole trisiloxane or mixtures thereof. Each sunscreen may be added at a concentration ranging from 0.001 to 20% by weight relative to the total weight of the composition.

The compositions according to the invention may additionally comprise any additive customarily used in the cosmetic or pharmaceutical field, such as sequestrants, antioxidants, preservatives, fillers, electrolytes, humectants, colourings, customary inorganic or organic bases or acids, perfumes, essential oils, cosmetic active agents, moisturizers, vitamins, essential fatty acids, sphingolipids, self-tanning compounds, skin soothing and protecting agents such as allantoin. Of course, persons skilled in the art will be careful to choose this or these optional additional compounds, and/or their quantity, such that the advantageous properties of the composition according to the invention are not, or not substantially, impaired.

These additives may be present in the composition in an amount of 0.001 to 20% by weight relative to the total weight of the composition.

There may be mentioned as example of sequestering agents: ethylenediaminetetraacetic acid (EDTA), and its derivatives or its salts, dihydroxyethylglycine, citric acid and tartaric acid.

There may be mentioned as examples of preservatives benzalkonium chloride, phenoxyethanol, benzyl alcohol, diazolidinylurea and parabens.

There may be mentioned as examples of humectants: glycerin and sorbitol.

The subject of the present invention is also the composition according to the invention as described above as a medicament.

The invention also relates to the use of the composition according to the invention as described above in the pharmaceutical and cosmetic field.

Compositions of the invention are particularly suitable for the treatment and prevention of hyperpigmentary disorders such as melasma, chloasma, lentigines, senile lentigo, vitiligo, freckles, post-inflammatory hyperpigmentations due to an abrasion, a burn, a scar, a dermatosis, a contact allergy; naevi, hyperpigmentations with a genetic determinism, hyperpigmentations of metabolic or drug origin, melanomas or any other hyperpigmentary lesions.

The compositions according to the invention also find application in the cosmetic field, in particular in protection against the harmful effects of the sun, for preventing and/or combating photoinduced or chronologic ageing of the skin and of the superficial body growths.

The invention also relates to a method for a nontherapeutic cosmetic treatment for beautifying the skin and/or for improving its surface appearance, characterized in that a composition comprising adapalene and at least one depigmenting agent is applied to the skin and/or its superficial body growths.

The examples of formulations below make it possible to illustrate the compositions according to the invention, without however limiting the scope thereof. Examples illustrating the depigmenting activity of the various compositions according to the invention are also described.

In the compositions below (Examples 1 to 5), the proportions of the various constituents are expressed as a percentage by weight relative to the total weight of the composition.

### Example 1:

| | |
|---|---|
| Adapalene | 0.1 |
| 4-Hydroxyanisole | 4 |
| Steareth-2 | 3 |
| Steareth-21 | 2 |
| Caprylic-capric triglyceride | 4 |
| Isohexadecane | 5 |
| Cetearyl alcohol | 1 |
| Stearic acid | 1.5 |
| Sodium sulphite | 0.2 |
| Propylene glycol | 8 |
| Glycerin | 2 |
| Phenoxyethanol | 1 |
| Citric acid (qs pH 5.5-6.0) | / |
| Purified water | qs 100 |

This composition should be applied twice per day until complete depigmentation is obtained for the treatment of lentigines.

### Example 2:

| | |
|---|---|
| Adapalene | 0.1 |
| Hydroquinone | 2 |
| Carbomer | 0.1 |
| Methyl Paraben | 0.18 |
| Propyl Paraben | 0.02 |
| Cetyl alcohol | 1 |
| Stearic acid | 0.8 |
| Caprylic-capric triglyceride | 1.5 |
| Cyclomethicone | 1 |
| Inorganic oil | 2 |
| Propylene glycol | 5 |
| Glycerin | 2 |
| Triethanolamine | 5 |
| Citric acid (qs pH 5.5-6.0) | / |
| Purified water | qs 100 |

This composition should be applied once per day until complete depigmentation is obtained for the treatment of melasma.

### Example 3:

| | |
|---|---|
| Adapalene | 0.10 |
| Hydroquinone | 4.00 |
| Magnesium and aluminium silicate | 3.00 |
| Butylated hydroxytoluene | 0.04 |
| Methyl Paraben | 0.18 |
| Propyl Paraben | 0.02 |
| Cetyl alcohol | 4.00 |
| Stearic acid | 3.00 |
| Stearyl alcohol | 4.00 |
| Glyceryl Stearate/PEG-100 stearate | 3.50 |
| Methyl Gluceth-10 | 5.00 |
| Glycerin | 4.00 |
| Citric acid | 0.05 |
| Sodium metabisulphite | 0.20 |
| Purified water | qs 100 |

This composition should be applied twice per day until complete depigmentation is obtained for the treatment of melasma.

### Example 4:

| | |
|---|---|
| Adapalene | 0.10 |
| Hydroquinone | 4.00 |
| Magnesium and aluminium silicate | 3.00 |
| Butylated hydroxytoluene | 0.04 |
| Methyl Paraben | 0.18 |
| Propyl Paraben | 0.02 |
| Cetyl alcohol | 4.00 |
| Stearic acid | 3.00 |
| Stearyl alcohol | 4.00 |
| Glyceryl Stearate/PEG-100 stearate | 3.50 |
| Avobenzone | 2.00 |
| Titanium dioxide | 2.00 |
| Methyl Gluceth-10 | 5.00 |
| Glycerin | 4.00 |
| Citric acid | 0.05 |
| Sodium metabisulphite | 0.20 |
| Purified water | qs 100 |

This composition should be applied twice per day until complete depigmentation is obtained for the treatment of melasma.

### Example 5:

| | |
|---|---|
| Adapalene | 0.10 |
| Hydroquinone | 4.00 |
| Magnesium and aluminium silicate | 3.00 |
| Butylated hydroxytoluene | 0.04 |
| Methyl Paraben | 0.18 |
| Propyl Paraben | 0.02 |
| Cetyl alcohol | 4.00 |
| Stearic acid | 3.00 |
| Stearyl alcohol | 4.00 |
| Glyceryl Stearate/PEG-100 stearate | 3.50 |
| Ethylhexyl methoxycinnamate | 2.00 |
| Methyl Gluceth-10 | 5.00 |
| Glycerin | 4.00 |
| Citric acid | 0.05 |
| Sodium metabisulphite | 0.20 |
| Ecamsule | 3.00 |
| Purified water | qs 100 |

This composition should be applied once per day until complete depigmentation is obtained for the treatment of chloasma.

### Example 6 - Measurement of the depigmenting activity of the combination of adapalene and a depigmenting agent

The evaluation of the depigmenting and/or anti-pigmenting activity of hydroquinone 3% and Adapalene 0.1% alone or in combination for 8 weeks is carried out on the tail of SKH HR2 mice which is irradiated or not with ultraviolet B. The tail of the SKH:HR2 mouse is naturally pigmented and this pigmentation increases under the effect of repeated UVB irradiation. The depigmenting activity is measured on the natural pigmentation after application of the test product to the tail of nonirradiated animals.

The anti-pigmenting activity is measured by the inhibition of the induction of the UV-induced pigmentation: the test product is applied to the tail of animals irradiated with UVB.

The treatment is carried out for 5 days per week for 8 weeks. 20 µl of test product, diluted in acetone, are applied to the tail in a deferred manner: hydroquinone in the morning and adapalene 4 h later. On the days for irradiation, the treatment is applied after irradiation.

The animals are irradiated 3 times per week for 8 weeks (Monday, Wednesday, Friday) at the dose of 90 mJ/cm² of UVB.

The evaluation is made by different clinical observations: once per week before irradiation, the pigmentation is evaluated by virtue of a score on a scale from 0 to 4. The distribution of the scores is the following:
- 0:: natural pigmentation
depigmentation scale: scores -1 to -4
- -1: light depigmentation
- -2:: moderate depigmentation
- -3:: marked depigmentation
- -4:: total depigmentation
pigmentation scale: scores 1 to 4
- 1: light pigmentation
- 2: moderate pigmentation
- 3: marked pigmentation
- 4: intense pigmentation

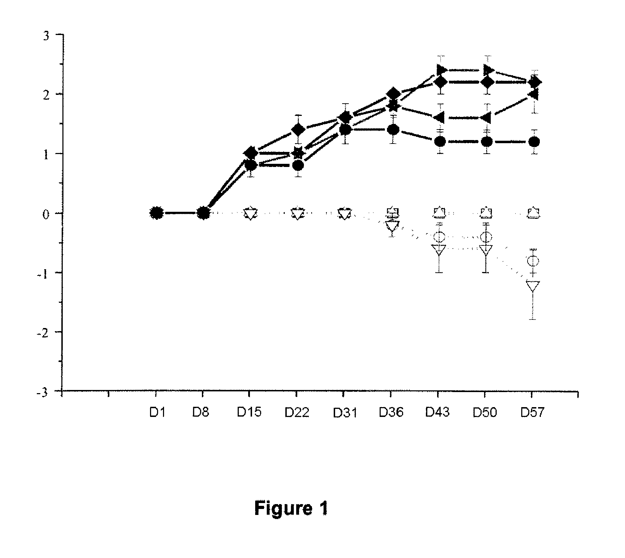

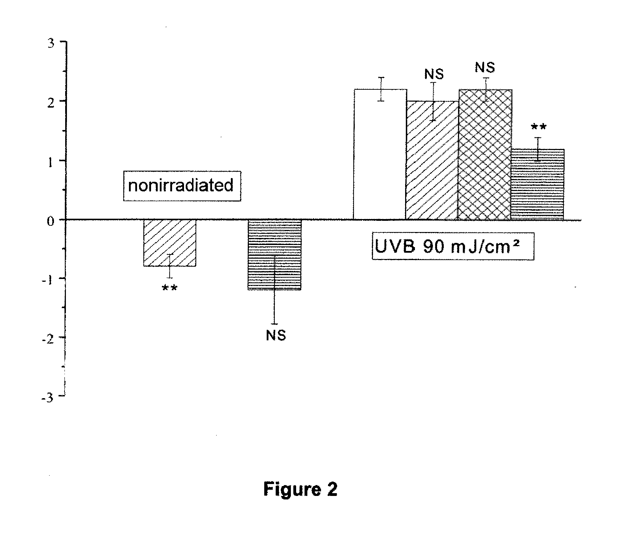

The results are represented in Figures 1 and 2.

Figure 1 represents the kinetics for the scores of pigmentation of the mouse skin as a function of the treatment time with or without irradiation with ultraviolet B (up to 8 weeks of treatment) with (◆) UVB + acetone, (►) UVB + Adapalene, (◄) UVB + hydroquinone, (•) UVB+hydroquinone+adapalene, [□] nonirradiated skin + acetone, (△) nonirridiated skin + adapalene (o) nonirradiated skin + hydroquinone (∇) nonirradiated skin + hydroquinone + adapalene.

Figure 2 represents the pigmentation scores at the end of the study (D57) with (□] acetone, ( 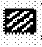 ) hydroquinone, ( 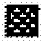 ) adapalene, ( 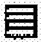 ) adapalene + hydroquinone with or without ultraviolet B irradiation. Depigmenting activity: hydroquinone alone at 3% induces a clinically visible depigmentation from the 6th week of treatment (D43) and a statistically significant depigmentation at the end of the study. Adapalene alone does not modify the natural pigmentation. When adapalene is combined with Hydroquinone, it potentiates its depigmenting activity.
Anti-pigmenting activity: in the animals irradiated and treated concomitantly, hydroquinone shows an anti-pigmenting effect at the 6th and at the 7th week (D50) which becomes less marked at the end of the study. On the other hand, the adapalene + hydroquinone combination inhibits the pigmentation induced by UVB irradiation from its appearance and up to the end of the study where the difference is statistically significant (^{**},p<0.01).

### Conclusion

After 8 weeks of topical treatment on the tail of SKH:HR2 mice, it is noted that the hydroquinone + adapalene combination exhibits a high antipigmenting activity and significantly inhibits the pigmentation induced by UVB irradiation from its appearance and up to the end of the study. The hydroquinone + adapalene combination has a significant benefit as depigmenting agent in relation to hydroquinone alone.

## Claims

1. Depigmenting composition for the skin, comprising adapalene and at least one depigmenting agent, with the exception of glycolic acid and azelaic acid, in a physiologically acceptable vehicle.

2. Composition according to Claim 1, **characterized in that** the depigmenting agent is chosen from phenolic derivatives, kojic acid and its derivatives, linoleic acid, resorcinol and its derivatives, ellagic acid, hydroxy acids, ascorbic acid and its derivatives, zinc peroxide, mercury chloride, arbutin and its derivatives, aminophenol derivatives, iminophenol derivatives, L-2-oxothiazolidine-4-carboxylic acid, its salts and esters, and extracts of liquorice, mulberry and skullcap.

3. Composition according to Claim 1 or 2, **characterized in that** the depigmenting agent is a phenolic derivative.

4. Composition according to any one of Claims 1 to 3, **characterized in that** the depigmenting agent is hydroquinone or 4-hydroxyanisole.

5. Composition according to any one of Claims 1 to 4, **characterized in that** it also contains at least one sunscreen.

6. Composition according to any one of Claims 1 to 5, **characterized in that** it additionally contains at least one cosmetic or pharmaceutical additive.

7. Composition according to any one of Claims 1 to 6, as a medicament.

8. Use of a depigmenting composition for the skin, comprising adapalene and at least one depigmenting agent in a physiologically acceptable vehicle for the manufacture of a pharmaceutical preparation intended for the prevention or treatment of hyperpigmentary disorders such as melasma, chloasma, lentigines, vitiligo, freckles, post-inflammatory hyperpigmentations due to an abrasion, a burn, a scar, a dermatosis, a contact allergy; naevi, hyperpigmentations with a genetic determinism, hyperpigmentations of metabolic or drug origin, melanomas or any other hyperpigmentary lesions.

9. Use according to Claim 8, for the manufacture of a pharmaceutical preparation intended for the prevention or treatment of melasma.

10. Cosmetic use of a depigmenting composition for the skin, comprising adapalene and at least one depigmenting agent in a physiologically acceptable vehicle, for protection against the harmful effects of the sun, for preventing and/or combating photoinduced or chronologic ageing of the skin and of the superficial body growths.

11. Method for a nontherapeutic cosmetic treatment for beautifying the skin and/or for improving its surface appearance, **characterized in that** a composition comprising adapalene and at least one depigmenting agent is applied to the skin and/or its superficial body growths.
